Europäisches Patentamt

European Patent Office

Office européen des brevets

(19)

(11) **EP 0 776 880 B1**

(12) **FASCICULE DE BREVET EUROPEEN**

(45) Date de publication et mention
de la délivrance du brevet:
**16.02.2000 Bulletin 2000/07**

(51) Int Cl.[7]: **C07C 45/50**, C07C 45/49

(21) Numéro de dépôt: **96402478.0**

(22) Date de dépôt: **18.11.1996**

(54) **Procédé pour l'hydroformylation des composés oléfiniques**

Verfahren zur Hydroformylierung von olefinischen Verbindungen

Process for the hydroformylation of olefinic compounds

(84) Etats contractants désignés:
**DE ES GB IT NL**

(30) Priorité: **30.11.1995 FR 9514147**

(43) Date de publication de la demande:
**04.06.1997 Bulletin 1997/23**

(73) Titulaire: **INSTITUT FRANCAIS DU PETROLE**
**92852 Rueil-Malmaison Cedex (FR)**

(72) Inventeurs:
- **Chauvin, Yves**
  **78230 Le Pecq (FR)**
- **Olivier, Hélène**
  **92500 Rueil Malmaison (FR)**
- **Mussmann, Lothar,**
  **63067 Offenbach (DE)**

(56) Documents cités:
**EP-A- 0 107 430**       **US-A- 3 511 880**
**US-A- 3 832 391**

## Description

L'objet de la présente invention est un nouveau procédé d'hydroformylation de composés oléfiniquement insaturés par de l'oxyde de carbone et de l'hydrogène dans lequel le système catalytique est une solution d'au moins un composé d'un métal de transition dans un sel ionique non aqueux organique-inorganique liquide à la température de réaction, et dans lequel les produits issus de la réaction d'hydroformylation sont peu ou pas solubles.

L'hydroformylation des composés oléfiniques est une réaction de grande importance industrielle et la plupart des procédés ont recours à des catalyseurs qui sont dissous dans une phase organique constituée des réactifs, des produits et éventuellement d'un excès de ligands, si bien que l'on rencontre des difficultés pour séparer et récupérer le catalyseur, en particulier quand celui-ci est un métal noble comme par exemple le rhodium.

Une solution en vue de résoudre ce problème a été décrite dans le brevet français FR-2.314.910. Elle consiste à effectuer l'hydroformylation en présence d'une solution aqueuse contenant un complexe du rhodium qui est rendu hydrosoluble grâce à la présence d'un ligand phosphine sulfonée lui-même hydrosoluble tel que le sel de sodium de la triphénylphosphine trisulfonée. De cette manière la phase organique contenant les aldéhydes est aisément séparée de la phase aqueuse contenant le catalyseur. Cette technique a fait l'objet d'un nombre considérable de travaux qui ont été discutés dans un article de W.A. Herrmann paru dans Angewandte Chemie International en 1993, volume 32, page 1524 et suivantes. En dépit du grand intérêt industriel de cette technique pour l'hydroformylation du propylène, ce système à deux phases souffre du manque de solubilité des oléfines dans l'eau, ce qui conduit à des vitesses de réaction relativement faibles qui la rend inapplicable pour les oléfines à longue chaîne.

Par ailleurs a été décrit dans le brevet U.S. 3.565.823 une technique consistant à disperser un composé d'un métal de transition dans un sel d'étain ou de germanium et d'un ammonium ou d'un phosphonium quaternaire, de formule $(R_1R_2R_3R_4Z)YX_3$ dans laquelle $R_1R_2,R_3,R_4$, sont des restes hydrocarbyles ayant jusqu'à 18 atomes de carbone, Z est l'azote ou le phosphore, Y l'étain ou le germanium et X un halogène, chlore ou brome, ce milieu non-aqueux à caractère ionique constituant un "sel fondu". Dans le brevet U.S. 3.657.368 a été décrit un procédé d'hydrogénation des oléfines et dans le brevet U.S. 3.919.271 un procédé d'hydrogénation des nitriles utilisant tous deux la composition précédente à base d'étain et de germanium. Dans le brevet U.S. 3.832.391 a été revendiqué un procédé de carbonylation des oléfines par la même composition.

La demande de brevet EP-A-107.430 décrit un procédé d'hydroformylation en présence d'un catalyseur au ruthénium dispersé dans un sel ou une base de phosphonium ou d'ammonium quaternaires présentant un point de fusion inférieur à la température de réaction.

Les anions de sel utilisés sont les halogénures, nitrate, acétate, chromate, hydroxyde. Le tétrafluoroborate de tétrabutylphosphonium peut également être employé.

Tous ces sels présentent des points de fusion d'au moins 100°C, et plus généralement d'au moins 120°C. De sorte que le catalyseur solide de ruthénium est mélangé au sel solide, et, en présence du réactif, l'ensemble est chauffé à la température de réaction où le milieu est liquide.

Les compositions précédemment décrites présentent le désavantage d'avoir un point de fusion relativement élevé, la réaction d'hydroformylation ayant alors lieu par exemple à au moins 90°C, ou généralement 160-180°C dans EP-A-107.430.

Il a maintenant été trouvé que l'on pouvait à la fois bénéficier des avantages d'une mise en oeuvre à deux phases tout en évitant les inconvénients liés d'une part à l'utilisation de l'eau et d'autre part à l'emploi des composés à point de fusion élevé, en dissolvant les composés catalytiques de métaux de transition des groupes 8, 9 et 10 et en particulier les composés du cobalt, du ruthénium, du rhodium, de l'iridium, du palladium et du platine, connus pour catalyser l'hydroformylation, dans des sels organiques-inorganiques liquides à basse température. Le sel agit alors comme un solvant du composé catalytique.

Plus précisément, l'invention a pour objet un procédé pour l'hydroformylation en phase liquide des composés oléfiniquement insaturés dans lequel la réaction est effectuée en présence d'au moins un sel organique-inorganique de formule générale $Q^+A^-$, dans laquelle $Q^+$ représente un ammonium quaternaire et/ou phosphonium quaternaire, et $A^-$ représente un anion, ledit sel ne contenant pas d'étain ou de germanium, et ledit sel étant liquide à une température inférieure à 90°C, et en présence d'au moins un composé d'un métal de transition des groupes 8, 9 et 10.

Les sels liquides selon l'invention ont pour formule générale $Q^+ A^-$ dans laquelle $Q^+$ représente un ammonium quaternaire et/ou d'un phosphonium quaternaire et $A^-$ représente tout anion connu comme étant non-coordinant susceptible de former un sel liquide à basse température, c'est-à-dire en-dessous de 90°C, et de préférence d'au plus 85° C, et de préférenceen dessous de 50°C tels de préférence les ions hexafluorophosphate, hexafluoroantimonate, hexafluoroarsénate, fluorosulfonate, tétrafluoroborate lorsque $Q^+$ représente l'ammonim, les bis-perfluoroalkylsulfonyl amides (en particuler les méthyl, butyl et nonyl), les perfluoroalkyl sulfonates (et en particulier le méthyl). On peut aussi utiliser les anions dichlorocuprate, tétrachloroborate, tétrachloroaluminate, trichlorozincate mais de façon non préférée. Les ammonium et/ou phosphonium quaternaires répondent de préférence aux formules générales $NR^1R^2R^3R^{4+}$ et $PR^1R^2R^3R^{4+}$, ou aux formules générales $R^1R^2N=CR^3R^{4+}$ et $R^1R^2P=CR^3R^4+$ où $R^1 R^2$, $R^3$ et $R^4$, identiques ou diffé-

rents, représentent l'hydrogène à l'exception du cation $NH_4+$ et de préférence un seul substituant peut représenter l'hydrogène, ou des restes hydrocarbyles ayant de 1 à 12 atomes de carbone, par exemple des groupements alkyles, saturés ou non saturés, cycloalkyles ou aromatiques, aryle ou aralkyle, comprenant de 1 à 12 atomes de carbone. Les ammonium et/ou phosphonium peuvent également être dérivés d'hétérocycles azotés ou phosphorés comportant 1, 2 ou 3 atomes d'azote et/ou de phosphore, de formule générales :

dans lesquelles les cycles sont constitués de 4 à 10 atomes, de préférence 5 à 6 atomes, $R^l$ et $R^2$ étant définis comme précédemment. L'ammonium ou le phosphonium quaternaire peuvent également être un cation de formule:

$$R^1R^{2+}N=CR^3-R^5-R^3C=N^+R^1R^2$$

$$R^1R^{2+}P=CR^3-R^5-R^3C=P^+R^1R^2$$

dans laquelle $R^1R^2,R^3$, identiques ou différents sont définis comme précédemment et $R^5$ représente un reste alkylène ou phénylène. Parmi les groupements $R^1,R^2$, $R^3$ et $R^4$ on mentionnera les radicaux méthyl, éthyl, propyl, isopropyl, butyl, secondaire butyl, tertiaire butyl, amyl, méthylène, éthylidène, phényl ou benzyl; $R^5$ pourra être un groupement méthylène, éthylène, propylène ou phénylène. Le cation ammonium et/ou phosphonium est choisi de préférence dans le groupe formé par le N-butylpyridinium, le N-éthylpyridinium, le butyl-3 méthyl-1 imidazolium, le diéthylpyrazolium, l'éthyl-3 méthyl-1 imidazolium, le pyridinium, le triméthylphénylammonium, le tétrabutylphosphonium. A titre d'exemples des sels utilisables selon l'invention on peut citer l'hexafluorophosphate de N-butylpyridinium, le tétrafluoroborate de N-éthyl pyridinium, le tétrafluoroborate de tétrabutylphosphonium, l'hexafluoroantimonate de butyl-3 méthyl-1 imidazolium, l'hexafluorophosphate de butyl-3 méthyl-1 imidazolium, le trifluorométhylsulfonate de butyl-3 méthyl-1 imidazolium , le fluorosulfonate de pyridinium, l'hexafluorophosphate de triméthylphénylammonium. Ces sels peuvent être utilisés seuls ou en mélange. Ils ont une fonction de solvant. Peuvent être également utilisés, par exemple, le dichlorocuprate de butyl-3-méthyl-1-imidazolium, le tétrachloroborate de pyridinium, le tétrachloroaluminate de butyl-3-méthyl-1-imidazolium, le trichlorozincate de butyl-3-méthyl-1-imidazolium.

Les composés des métaux de transition utilisables selon l'invention sont de façon générale tous les composés des métaux de transition des groupes 8, 9 et 10 et notamment ceux connus de l'homme de l'art pour hydroformyler les oléfines. Ils peuvent être utilisés seuls ou en mélange. Ils peuvent être complexés ou associés à un ligand organique. Ils peuvent être mis en oeuvre sous forme de sels, et de façon préférée, qui n'est pas un halogénure. Il s'agit entre autres des composés du cobalt, du rhodium, de l'iridium, du ruthénium, du palladium et du platine. On peut avantageusement associer à tous ces composés des ligands organiques tels que les phosphines, arsines et stibines tertiaires, les phosphites, en particulier les arylphosphites. Ils peuvent être mono- ou bidentés. Ces ligands peuvent porter sur l'hétéroatome et/ou sur la chaîne carbonée, en outre au moins une autre fonction telle que amine, ammonium, alcool, acide carboxylique, sulfonate. A titre d'exemples on peut citer la triphénylphosphine, l'oxyde de triphénylphosphine, le triphénylphosphite, le triméthylphosphite, le sel de sodium de la triphényl phosphine monosulfonée, le sel de sodium de la triphényl phosphine trisulfonée. Le choix du composé catalytique du métal de transition n'est pas critique. On mentionnera, par exemple, $HRh(CO)(PR_3)_3$, $HRh(CO)_2(PR_3)$, $HRh(CO)[P(OR)_3]_3$, $Rh(acac)(CO)_2$, (acac signifiant acétylacétonate) $Rh_6(CO)_{16}$, $[Rh(norbornadiène)(PPh_3)_2]^+[PF_6]^-$, $[Rh(CO)_3(PPh_3)_2]^+[BPh_4]^-$, $RhCl(CO)(PEt_3)_2$, $[RhCl(cyclooctadiène)]_2$, $[Rh(CO)_3(PR_3)_2]^+BPh^{4-}$, $[Rh(CO)_3(PR_3)_2]^+PF_6^-$, $HCo(CO)_4$, $Ru_3(CO)_{12}$, $[RuH(CO)(acétonitrile)_2(PPh_3)_3]^+[BF_4]^-$, $PtCl_2(cyclooctadiène)$, $[Ir(CO)_3(PPh_3)]^+[PF_6]^-$, $[HPt(PEt_3)_3]^+[PF_6]$, avec R est un radical hydrocarbyl, par exemple alkyl, cycloalkyl, aryl, substitués ou non. Mais on peut aussi avoir recours à des sels totalement inorganiques, précurseurs de catalyseur, tels que $Rh_2O_3$ , $Pd(NO_3)_2$ et $Rh(NO_3)_3$, et de façon non préférée à des halogénures tel que $RhCl_3,3H_2O$. On peut également dissoudre préalablement le composé du métal de transition et/ou le ligand dans un solvant organique.

La composition catalytique est obtenue par mélange, d'une manière quelconque, du sel liquide avec le composé du métal de transition et éventuellement le ligand.

Les complexes catalytiques métal de transition-ligand organique peuvent être préparés en dehors du milieu réactionnel et y être introduits pour la réaction. Ils peuvent également être formés in situ, dans le milieu réactionnel, par introduction des composants nécessaires à leur formation.

Un autre avantage lié au procédé de la présente invention réside en ce que on peut utiliser une très grande variété de ligands qui ne sont pas compatibles avec l'eau mais qui sont stables dans ces milieux, comme par exemple les phosphites qui sont très facilement hydrolysables et dont la synthèse est bien plus aisée que celle des phosphines. D'une façon générale, la composition catalytique peut contenir un solvant organique miscible ou partiellement miscible comme un hydrocarbure aromatique, et/ou un hydrocarbure aliphatique non miscible qui permet une meilleure séparation des phases. De façon préférée, la composition catalytique ne contient pas d'eau.

La concentration du composé (du complexe, de préférence) de métal de transition dans le "sel fondu" n'est pas critique. Elle est avantageusement comprise entre 1 mmole de composé par litre de "sel fondu" et 500 mmoles par litre, de préférence entre 2 et 200 mmoles par litre, et encore entre 2 et 100, voire 2 à 50. Le rapport molaire entre le ligand organique et le composé du métal de transition peut être compris entre 1 et 100, de préférence entre 1 et 20.

Les composants entrant dans la composition selon l'invention, peuvent être mélangés dans un ordre quelconque, à une température comprise entre -20°C et + 200°C, avantageusement entre -20°C et moins de 90°C, et de préférence 30°C à moins de 150°C et avantageusement de 0°C à moins de 150°C, 0°C à 120°C, ou encore 0 - moins de 90°C, de préférence 0 - 85°C ou 0 à 50°C.

Les composés oléfiniquement insaturés susceptibles d'être hydroformylés selon l'invention sont les monooléfines, les dioléfines et en particulier les dioléfines conjuguées, des composés comportant un ou des hétéroatomes notamment insaturés comme la fonction cétone ou acide carboxylique. A titre d'exemple on peut citer l'hydroformylation de l'éthylène en propionaldéhyde, du propylène en butyradéhyde et isobutyradéhyde, du butène en pentanal et isopentanals, du pentène en hexanal et isohexanals, des hexènes en isoheptanals, des isooctènes en isononanals, le butadiène en adipaldéhyde ou encore l'hydroformylation des octènes, pentadiènes. Ces composés peuvent être utilisés purs ou dilués par des hydrocarbures saturés ou insaturés.

Le rapport des pressions partielles du monoxyde de carbone a l'hydrogène utilisé dans le milieu réactionnel pour l'hydroformylation peut être de 1:10 à 10:1, de préférence dans un rapport 1:1, mais tout autre rapport peut être utilisé selon la mise en oeuvre du procédé.

La température à laquelle se fera l'hydroformylation sera comprise entre 30°C et 200°C, avantageusement la température est inférieure à 150°C, de préférence entre 50°C et moins de 150°C de préférence inférieure à 90°C, et encore plus avantageusement d'au plus 85°C. Une gamme de températures préférées est entre 50°C et moins de 150°C et encore plus avantageusement de 30°C à 120°C, de 30°C à moins de 90°C. La pression peut être comprise entre 1 MPa et 20 MPa, de préférence entre la 2MPa et 10 MPa.

La réaction catalytique d'hydroformylation des composés insaturés peut être conduite en système fermé, en système semi-ouvert ou en continu avec un ou plusieurs étages de réaction. A la sortie du réacteur la phase organique contenant les produits de réaction (aldéhydes) est séparée avantageusement par simple décantation de la phase polaire catalytique contenant le "sel fondu" et la majeure partie du catalyseur. La phase polaire qui contient au moins en partie le catalyseur, est, au moins en partie, retournée au réacteur, l'autre partie étant traitée pour éliminer les résidus du catalyseur.

Les exemples suivants illustrent l'invention sans en limiter la portée:

EXEMPLE 1

Dans un réacteur en acier inoxydable à double enveloppe d'une contenance de 100 mL purgé de l'air et de l'humidité et placé sous la pression atmosphérique du mélange (1:1 mol/mol) hydrogène-oxyde de carbone, on a introduit à température ambiante 4 mL d'hexafluorophosphate de butylméthylimidazolium liquide, 19,3 mg (0.075 mmole) du complexe Rh(acétylacétonate)(CO)$_2$ et 186 mg (0,71 mmole) de triphénylphosphine dissous dans 2 mL de toluène, 2 mL d'heptane (étalon) et 7,5 mL (68 mmole) de 1-pentène. On a porté la pression du mélange hydrogène-oxyde de carbone à 2 MPa et la température à 82°C et on a mis l'agitation en route. Après 2 heures on a arrêté l'agitation et on a laissé se décanter le mélange; on a soutiré la phase organique surnageante qui était très légèrement colorée. La conversion du 1-pentène était de plus de 99 %. Le rendement molaire était de 75% en hexanal et de 24% en 2-méthylpentanal. Le reste (1 %) était constitué de 2-pentène et de pentane.

EXEMPLE 2

On a repris la phase ionique de l'essai précédent de laquelle avait été décantée la phase organique et on y a ajouté 7,5 mL de 1-pentène et 2 mL d'heptane. On a placé ce mélange sous pression du mélange hydrogène-oxyde de carbone dans le mêmes conditions que celles de l'essai précédent. Après 2 heures de réaction la conversion du 1-pentène était de plus de 99 % et la distribution des produits pratiquement identique à celle de l'exemple 1.

EXEMPLE 3

On a opéré dans l'appareil décrit dans l'exemple 1. On y a introduit à température ambiante 4 mL de tétrafluoroborate d'éthylméthylimidazolium liquide, 0,5 mL d'eau, 19,3 mg (0.075 mmole) du complexe Rh(acétylacétonate)(CO)$_2$, 0,71 mmole du sel de sodium de la triphénylphosphine trisulfonée, 2 mL d'heptane (étalon) et 7,5 mL (68 mmole) de 1-pentène. On a placé le tout sous 4 MPa du mélange oxyde de carbone-hydrogène et on a porté la température à 80°C. Après 2 heures de réaction, on a décanté la phase organique qui était parfaitement incolore. La conversion du 1-pentène était de 33%, et la sélectivité en aldéhydes supérieure à 99%.

EXEMPLE 4

On a opéré dans l'appareil décrit dans l'exemple 1. On y a introduit 4 mL de tétrafluoroborate d'éthylméthylimidazolium, 0,5 mL d'eau, 0.075 mmole du complexe PtCl$_2$(cyclooctadiène), 0,71 mmole du sel de sodium de la triphénylphosphine trisulfonée, 2 mL d'heptane (étalon) et 7,5 mL (68 mmole) de 1-pentène. On a placé le tout sous 4 MPa du mélange oxyde de carbone-hydrogène et on a porté la température à 80°C. Après 2 heures de réaction, on a décanté la phase organique qui était parfaitement incolore. La conversion du 1-pentène était supérieure à 25%, et la sélectivité en aldéhydes supérieure à 99%.

EXEMPLE 5

On a opéré dans l'appareil décrit dans l'exemple 1. On y a introduit 4 mL de tétrafluoroborate d'éthylméthylimidazolium, 0,5 mL d'eau, 0.075 mmole du complexe $[RuH(CO)(acétonitrile)_2(PPh_3)_3]^+[BF_4]^-$, 2 mL d'heptane (étalon) et 7,5 mL (68 mmole) de 1-pentène. On a placé le tout sous 4 MPa du mélange oxyde de carbone-hydrogène et on a porté la température à 80°C. Après 2 heures de réaction, on a décanté la phase organique qui était parfaitement incolore. La conversion du 1-pentène était supérieure à 25%, et la sélectivité en aldéhydes supérieure à 99%.
Le procédé selon l'invention présente donc, outre l'avantage de pouvoir faire la réaction à des températures inférieures à celles de l'art antérieur si l'exploitant le souhaite, de produire des aldéhydes avec une sélectivité élevée (au moins 40 % ou mieux au moins 50 % et plus généralement au moins 70 % voire même au moins 80 ou 85 %). Le fait que le sel fondu soit liquide à des températures voisines de l'ambiante ou même plus basses facilite la mise en oeuvre du procédé, la manipulation de solides tel que dans l'art antérieur rend ces procédés plus difficiles à exploiter

**Revendications**

1.  Procédé d'hydroformylation en phase liquide d'au moins un composé oléfiniquement insaturé par de l'oxyde de carbone et de l'hydrogène en présence d'une composition catalytique contenant au moins un composé d'un métal de transition et au moins un sel organique inorganique ne contenant pas d'étain ou de germanium procédé caractérisé en ce que ledit sel est un sel d'ammonium et/ou phosphonium quaternaire de formule générale $Q^+A^-$ dans laquelle $Q^+$ représente un ammonium et/ou un phosphonium quaternaire, et $A^-$ représente un anion, et que ledit sel est liquide à une température inférieure à 90 °C.

2.  Procédé selon la revendication 1 dans lequel l'anion $A^-$ est choisi dans le groupe formé par l'hexafluorophosphate, l'hexafluoroantimonate, l'hexafluoroarsénate, le fluorosulfonate, le tétrafluoroborate lorsque $Q^+$ représente l'ammonium, les bis-perfluoro-alkylsulfonyl amides, les perfluoroalkyl-sulfonates ou bien encore le dichlorocuprate, le tétrachloroborate, le tétrachloroaluminate, le trichlorozincate.

3.  Procédé selon l'une des revendications 1 ou 2 dans lequel le cation ammonium et/ou phosphonium quaternaires sont choisis dans le groupe formé par les cations de formule générale:

$$R^1R^2R^3R^4N^+$$

$$R^1R^2N=CR^3R^{4+}$$

$$R^1R^2R^3R^4P^+$$

$$R^1R^2P=CR^3R^{4+}$$

dans lesquelles $R^1, R^2, R^3, R^4$, identiques ou différents représentent l'hydrogène, à l'exception de $NH_4^+$, et des restes hydrocarbyles ayant 1 à 12 atomes de carbone, et dans lesquelles les cycles sont constitués de 4 à 10 atomes.

**4.** Procédé selon l'une des revendications 1 ou 2 dans laquelle le cation ammonium et/ou phosphonium quaternaires ont pour formules générales:

$$R^1R^{2+}N=CR^3-R^5-R^3C=N^+R^1R^2$$

$$R^1R^{2+}P=CR^3-R^5-R^3C=P^+R^1R^2$$

dans laquelle $R^1R^2, R^3$ identiques ou différents représentent l'hydrogène ou des restes hydrocarbyles ayant 1 à 12 atomes de carbone, et $R^5$ représente un reste alkylène ou phénylène.

**5.** Procédé selon l'une des revendications précédentes dans lequel le cation ammonium et/ou phosphonium quaternaires sont choisis dans le groupe constitué par le N-butylpyridinium, le N-éthylpyridinium, le butyl-3 méthyl-1 imidazolium, le diéthylpyrazolium, l'éthyl-3 méthyl-1 imidazolium, le pyridinium, le triméthylphénylammonium, le tétrabutylphosphonium.

**6.** Procédé selon l'une des revendications précédentes dans lequel les sels d'ammonium et/ou de phosphonium quaternaires sont choisis dans le groupe constitué par l'hexafluorophosphate de N-butylpyridinium, le tétrafluoroborate de N-éthyl pyridinium, le tétrafluoroborate de tétrabutylphosphonium, l'hexafluoroantimonate de butyl-3 méthyl-1 imidazolium, l'hexafluorophosphate de butyl-3 méthyl-1 imidazolium, le trifluoro-méthylsulfonate de butyl-3 méthyl-1 imidazolium , le fluorosulfonate de pyridinium, l'hexafluorophosphate de triméthylphénylammonium, le dichlorocuprate de butyl-3 méthyl-1 imidazolium, le tétrachloroborate de pyridinium, le tétrachloroaluminate de butyl-3 méthyl-1 imidazolium, le trichlorozincate de butyl-3 méthyl-1 imidazolium.

**7.** Procédé selon l'une des revendications précédentes dans lequel le métal de transition est le cobalt, le rhodium, l'iridium, le ruthénium, le palladium et le platine.

**8.** Procédé selon les revendications précédentes dans lequel le composé de métal de transition est un complexe de métal de transition.

**9.** Procédé selon l'une des revendications précédentes dans lequel la composition catalytique contient également un ligand ou un ligand associé au composé du métal de transition, ledit ligand étant choisi dans le groupe formé par les phosphines tertiaires, les arsines tertiaires, les stibines tertiaires, les phosphites.

**10.** Procédé selon l'une des revendications précédentes dans lequel le composé du métal de transition est choisi dans le groupe formé par $HRh(CO)(PR_3)_3$, $HRh(CO)_2(PR_3)$, $HRh(CO)[P(OR)_3]_3$, $.Rh(acac)(CO)_2$, (acac signifiant acétylacétonate) $Rh_6(CO)_{16}$, $[Rh(CO)_3(PPh_3)_2]^+[BPh_4]^-$, $RhCl(CO)(PEt_3)_2$, $[RhCl(cyclooctadiène)]_2$, $[Rh(CO)_3(PR_3)_2]^+BPh_4^-$, $[Rh(CO)_3(PR_3)_2]^+PF_6^-$, $[Rh(norbornadiène)(PPh_3)_2]^+[PF_6]^-$, $HCo(CO)_4$, $Ru_3(CO)_{12}$, $[RuH(CO)$

# EP 0 776 880 B1

(acétonitrile)$_2$(PPh$_3$)$_3$]$^+$[BF$_4$]$^-$, PtCl$_2$ (cy-clooctadiène), [Ir(CO)$_3$(PPh$_3$)]$^+$[PF$_6$]$^-$, [HPt(PEt$_3$)$_3$]$^+$[PF$_6$].$^-$, Rh$_2$O$_3$, Pd(NO$_3$)$_2$ et Rh(NO$_3$)$_3$, avec R radical hydrocabyl substitué ou non.

11. Procédé selon la revendication 9 dans lequel le ligand contient au moins une fonction amine, ammonium, alcool, acide carboxylique ou sulfonate.

12. Procédé selon l'une des revendications précédentes dans lequel la composition catalytique contient un ligand ou un ligand associé au composé du métal de transition choisi dans le groupe formé par la triphénylphosphine, l'oxyde de triphénylphosphine, le triphénylphosphite, la triéthylphosphite, le sel de sodium de la triphénylphosphine monosulfonée, le sel de sodium de la triphénylphosphine trisulfonée.

13. Procédé selon l'une des revendications précédentes dans lequel la concentration du ou des composés du ou des métaux de transition par rapport au sel d'ammonium et/ou de phosphonium est de 1 à 500 mmoles par litre.

14. Procédé selon l'une des revendications précédentes dans lequel la composition catalytique contient également un solvant organique.

15. Procédé selon la revendications 14 dans lequel le solvant est choisi dans le groupe formé par les hydrocarbures aromatiques et les hydrocarbures aliphatiques.

16. Procédé selon l'une des revendications précédentes dans lequel le composé oléfiniquement insaturé est choisi dans le groupe formé par les monooléfines, les dioléfines, les dioléfines conjuguées, les composés comportant un ou des hétéroatomes insaturés.

17. Procédé selon la revendication 16 dans lequel la monooléfine est choisie dans le groupe formé par l'éthylène, le propylène, le butène, le pentène, les hexènes, les octènes et la dioléfine dans le groupe formé par le butadiène et les pentadiènes.

18. Procédé selon l'une des revendications 8 à 15 dans lequel le composé oléfiniquement insaturé est un composé portant une fonction cétone ou une fonction acide carboxylique.

19. Procédé selon l'une des revendications 1 à 16 dans lequel la phase organique contenant les produits de réaction est séparée de la phase polaire, ladite phase polaire contenant au moins une partie du catalyseur étant au moins en partie recyclée dans le réacteur d'hydroformylation.

20. Procédé selon l'une des revendications 1 à 18 opérant entre 30 et 200°C, sous une pression totale comprise entre 1 MPa et 20 MPa, les rapports des pressions partielles de l'oxyde de carbone à l'hydrogène étant de 1:10 à 10:1.

21. Procédé selon l'une des revendications 1 à 18 dans lequel les aldéhydes sont sélectivement obtenus.

22. Procédé selon l'une des revendications précédentes dans lequel l'hydroformylation est réalisée à une température inférieure à 90°C.

## Patentansprüche

1. Verfahren zur Hydroformylierung in flüssiger Phase mindestens einer olefinisch ungesättigten Verbindung mit Kohlenmonoxid und Wasserstoff in Gegenwart einer katalytischen Zusammensetzung, enthaltend mindestens eine Verbindung eines Übergangsmetalls und mindestens ein organischanorganisches Salz, das kein Zinn oder Germanium enthält, dadurch gekennzeichnet, daß das genannte Salz ein quaternäres Ammonium- und/oder Phosphoniumsalz der allgemeinen Formel Q$^+$A$^-$ ist, in der Q$^+$ für quaternäres Ammonium und/oder quaternäres Phosphonium und A$^-$ für ein Anion stehen, und daß das genannte Salz bei einer Temperatur unterhalb 90°C flüssig ist.

2. Verfahren nach Anspruch 1, bei dem das Anion A$^-$ ausgewählt wird aus der Gruppe, die besteht aus Hexafluorophosphat, Hexafluoroantimonat, Hexafluoroarsenat, Fluorosulfonat, Tetrafluoroborat, wenn Q$^+$ für Ammonium steht, den Bis-perfluoroalkylsulfonylamiden, den Perfluoroalkyl-sulfonaten oder auch Dichlorocuprat, Tetrachloroborat, Tetrachloroaluminat und Trichlorozinkat.

7

3. Verfahren nach einem der Ansprüche 1 oder 2, bei dem das quaternäre Ammonium- und/oder Phosphoniumkation ausgewählt aus wird (werden) aus der Gruppe, die besteht aus Kationen der allgemeinen Formel:

$$R^1R^2R^3R^4N^+$$

$$R^1R^2N=CR^3R^{4+}$$

$$R^1R^2R^3R^4P^+$$

$$R^1R^2P=CR^3R^{4+}$$

worin $R^1$, $R^2$, $R^3$, $R^4$, die gleich oder voneinander verschieden sind, Wasserstoff, mit Ausnahme von $NH_4^+$, und Hydrocarbyl-Reste mit 1 bis 12 Kohlenstoffatomen bedeuten und in denen die Ringe aus 4 bis 10 Kohlenstoffatomen bestehen.

4. Verfahren nach einem der Ansprüche 1 oder 2, bei dem das quaternäre Ammonium- und/oder Phosphonium-Kation die allgemeinen Formeln hat (haben):

$$R^1R^{2+}N=CR^3-R^5-R^3C=N^+R^1R^2$$

$$R^1R^{2+}P=CR^3-R^5-R^3C=P^+R^1R^2$$

worin $R^1$, $R^2$, $R^3$, die gleich oder voneinander verschieden sind, Wasserstoff oder Hydrocarbyl-Reste mit 1 bis 12 Kohlenstoffatomen und $R^5$ einen Alkylen- oder Phenylen-Rest bedeuten.

5. Verfahren nach einem der vorhergehenden Ansprüche, bei dem das quaternäre Ammonium- und/oder Phosphonium-Kation ausgewählt wird (werden) aus der Gruppe, die besteht aus N-Butylpyridinium, N-Ethylpyridinium, 3-Butyl-1-methyl-imidazolium, Diethylpyrazolium, 3-Ethyl-1-methylimidazolium, Pyridinium, Trimethylphenylammonium und Tetrabutylphosphonium.

6. Verfahren nach einem der vorhergehenden Ansprüche, bei dem die quaternären Ammonium- und/oder Phosphonium-Salze ausgewählt werden aus der Gruppe, die besteht aus N-Butylpyridinium-hexafluorophosphat, N-Ethylpyridinium-tetrafluoroborat, Tetrabutylphosphonium-tetrafluoroborat, 3-Butyl-1-methyl-imidazolium-hexafluoroantimonat, 3-Butyl-1-methyl-imidazolium-hexafluorophosphat, 3-Butyl-1-methyl-imidazolium-trifluoromethylsulfonat, Pyridinium-fluorosulfonat, Trimethylphenylammonium-hexafluorophosphat, 3-Butyl-1-methyl-imidazolium-dichlorocuprat, Pyridinium-tetrachloroborat, 3-Butyl-1-methyl-imidazolium-tetrachloroaluminat und 3-Butyl-1-methylimidazolium-trichlorozinkat.

7. Verfahren nach einem der vorhergehenden Ansprüche, bei dem das Übergangsmetall Kobalt, Rhodium, Iridium, Ruthenium, Palladium und Platin ist.

8. Verfahren nach einem der vorhergehenden Ansprüche, bei dem die Verbindung eines Übergangsmetalls ein Komplex eines Übergangsmetalls ist.

9. Verfahren nach einem der vorhergehenden Ansprüche, bei dem die katalytische Zusammensetzung außerdem einen Liganden oder einen mit der Verbindung des Übergangsmetalls assoziierten Liganden enthält, der ausgewählt wird aus der Gruppe, die besteht aus tertiären Phosphinen, tertiären Arsinen, tertiären Stibinen und Phosphiten.

10. Verfahren nach einem der vorhergehenden Ansprüche, bei dem die Verbindung des Übergangsmetalls ausgewählt wird aus der Gruppe, die besteht aus $HRh(CO)(PR_3)_3$, $HRh(CO)_2(PR_3)$, $HRh(CO)[P(OR)_3]_3$, $Rh(Acac)(CO)_2$, (Acac steht für Acetylacetonat), $Rh_6(CO)_{16}$, $[Rh(CO)_3(PPh_3)_2]^+[BPh_4]^-$, $RhCl(CO)(PEt_3)_2$, $[RhCl(Cyclooctadien)]_2$, $[Rh(CO)3(PR_3)_2]^+BPh_4^-$, $[Rh(CO)_3(PR_3)_2]^+PF_6^-$, $[Rh(Norbornadien)(PPh_3)_2]^+[PF_6]^-$, $HCo(CO)_4$, $Ru_3(CO)_{12}$, $[RuH(CO)(Acetonitril)_2(PPh_3)_3]^+[BF_4]^-$, $PtCl_2(Cyclooctadien)$, $[Ir(CO)_3(PPh_3)]^+[PF_6]^-$, $[HPt(PEt_3)_3]^+[PF_6]^-$, $Rh_2O_3$, $Pd(NO_3)_2$ und $Rh(NO_3)_3$, worin R einen substituierten oder unsubstituierten Hydrocarbylrest bedeutet.

11. Verfahren nach Anspruch 9, bei dem der Ligand mindestens eine Amin-, Ammonium-, Alkohol-, Carbonsäure- oder Sulfonat-Funktion enthält.

12. Verfahren nach einem der vorhergehenden Ansprüche, bei dem die katalytische Zusammensetzung einen Liganden oder einen mit der Verbindung des Übergangsmetalls assoziierten Liganden enthält, der ausgewählt wird aus der Gruppe, die besteht aus Triphenylphosphin, Triphenylphosphinoxid, Triphenylphosphit, Triethylphosphit, dem Natriumsalz von monosulfoniertem Triphenylphosphin und dem Natriumsalz von trisulfoniertem Triphenylphosphin.

13. Verfahren nach einem der vorhergehenden Ansprüche, bei dem die Konzentration der Verbindung(en) des Übergangsmetalls (der Übergangsmetalle), bezogen auf das Ammonium- und/oder Phosphoniumsalz, 1 bis 500 mmol pro Liter beträgt.

14. Verfahren nach einem der vorhergehenden Ansprüche, bei dem die katalytische Zusammensetzung außerdem ein organisches Lösungsmittel enthält.

15. Verfahren nach Anspruch 14, bei dem das Lösungsmittel ausgewählt wird aus der Gruppe, die besteht aus aromatischen Kohlenwasserstoffen und aliphatischen Kohlenwasserstoffen.

16. Verfahren nach einem der vorhergehenden Ansprüche, bei dem die olefinisch ungesättigte Verbindung ausgewählt wird aus der Gruppe, die besteht aus Monoolefinen, Diolefinen, konjugierten Diolefinen und ungesättigten Verbindungen, die ein oder mehrere Heteroatome enthalten.

17. Verfahren nach Anspruch 16, bei dem das Monoolefin ausgewählt wird aus der Gruppe, die besteht aus Ethylen, Propylen, Buten, Penten, den Hexenen und Octenen, und das Diolefin ausgewählt wird aus der Gruppe, die besteht aus Butadien und den Pentadienen.

18. Verfahren nach einem der Ansprüche 8 bis 15, bei dem die olefinisch ungesättigte Verbindung eine Verbindung ist, die eine Keton-Funktion oder eine Carbonsäure-Funktion trägt.

19. Verfahren nach einem der Ansprüche 1 bis 16, bei dem die organische Phase, welche die Reaktionsprodukte enthält, von der polaren Phase abgetrennt wird, die mindestens einen Teil des Katalysators enthält, der mindestens zum Teil in den Hydroformylierungs-Reaktor im Kreislauf zurückgeführt wird.

20. Verfahren nach einem der Ansprüche 1 bis 18, das zwischen 30 und 200°C unter einem Gesamtdruck zwischen 1MPa und 20 MPa durchgeführt wird, wobei die Verhältnisse zwischen den Kohlenmonoxid- und Wasserstoff-Partialdrucken 1:10 bis 10:1 betragen.

21. Verfahren nach einem der Ansprüche 1 bis 18, bei dem die Aldehyde selektiv erhalten werden.

22. Verfahren nach einem der vorhergehenden Ansprüche, bei dem die Hydroformylierung bei einer Temperatur unterhalb 90°C durchgeführt wird.

**Claims**

1. A process for liquid phase hydroformylation of at least one olefinically unsaturated compound by carbon monoxide and hydrogen in the presence of a catalytic composition containing at least one transition metal compound and at least one organic-inorganic salt which does not contain tin or germanium, the process being characterized in that said salt is a quaternary ammonium and/or phosphonium salt with general formula $Q^+A^-$, where $Q^+$ represents a quaternary ammonium and/or phosphonium, and $A^-$ represents an anion, and in that said salt is liquid at a temperature below 90 °C.

2. A process according to claim 1, in which anion $A^-$ is selected from the group formed by hexafluorophosphate, hexafluoroantimonate, hexafluoroarsenate, fluorosulphonate, tetrafluoroborate when $Q^+$. is ammonium, bis-perfluoroalkylsulphonyl amides, perfluoroalkyl sulphonates, also dichlorocuprate, tetrachloroborate, tetrachloroaluminate and trichlorozincate

3. A process according to claim 1 or claim 2, in which the quaternary ammonium and/or phosphonium cation is selected from the group formed by cations with general formula:

$$R^1R^2R^3R^4N^+$$

$$R^1R^2N=CR^3R^{4+}$$

$$R^1R^2R^3R^4P^+$$

$$R^1R^2P=CR^3R^{4+}$$

where $R^1$, $R^2$, $R^3$ and $R^4$, which may be identical or different, represent hydrogen with the exception of $NH_4^+$, and hydrocarbyl residues containing 1 to 12 carbon atoms, and in which the cycles are constituted by 4 to 10 atoms.

4. A process according to claim 1 or claim 2, in which the quaternary ammonium and/or phosphonium cations have general formulae:

$$R^1R^{2+}N=CR^3-R^5-R^3C=N+R^1R^2$$

$$R^1R^{2+}P=CR^3-R^5-R^3C=P+R^1R^2$$

where $R^1$, $R^2$, $R^3$, which may be identical or different, represent hydrogen or hydrocarbyl residues containing 1 to 12 carbon atoms, and $R^5$ represents an alkylene or phenylene residue.

5. A process according to any one of the preceding claims, in which the quaternary ammonium and/or phosphonium cations are selected from the group constituted by N-butylpyridinium, N-ethylpyridinium, 3-butyl-1-methylimidazolium, diethylpyrazolium, 3-ethyl-1-methylimidazolium, pyridinium, trimethylphenylammonium, and tetrabutylphosphonium.

6. A process according to any one of the preceding claims, in which the quaternary ammonium and/or phosphonium salts are selected from the group constituted by N-butylpyridinium hexafluorophosphate, N-ethylpyridinium tetrafluoroborate, tetrabutylphosphonium tetrafluoroborate, 3-butyl-1-methylimidazolium hexafluoroantimonate, 3-butyl-1-methylimidazolium hexafluorophosphate, 3-butyl-1-methylimidazolium trifluoromethylsulphonate, pyridinium fluorosulphonate, trimethylphenylammomium hexafluorophosphate, 3-butyl-1-methylimidazolium dichlorocuprate, pyridinium tetrachloroborate, 3-butyl-1-methylimidazolium tetrachloroaluminate, and 3-butyl-1-methylimidazolium trichlorozincate.

7. A process according to any one of the preceding claims, in which the transition metal is cobalt, rhodium, iridium, ruthenium, palladium or platinum.

8. A process according to any one of the preceding claims, in which the transition metal compound is a transition metal complex.

9. A process according to any one of the preceding claims, in which the catalytic composition also contains a ligand or a ligand associated with the transition metal compound, said ligand being selected from the group formed by tertiary phosphines, tertiary arsines, tertiary stibines and phosphites.

10. A process according to any one of the preceding claims, in which the transition metal compound is selected from the group formed by $HRh(CO)(PR_3)_3$, $HRh(CO)_2(PR_3)$, $HRh(CO)[P(OR)_3]_3$, $Rh(acac)(CO)_2$, (acac represents acetylaccetonate), $Rh_6(CO)_{16}$, $[Rh(CO)_3(PPh_3)_2]^+[BPh_4]^-$, $RhCl(CO)(PEt_3)_2$, $[RhCl(cyclooctadiene)]_2$, $[Rh(CO)_3(PR_3)_2]^+BPh_4^-$, $[Rh(CO)_3(PR_3)_2]^+PF_6^-$, $[Rh(norbornadiene)(PPh_3)_2]^+[PF_6]^-$, $HCo(CO)_4$, $Ru_3(CO)_{12}$, $[RuH(CO)(acetonitrile)_2(PPh_3)_3]^+[BF_4]^-$, $PtCl_2(cyclooctadiene)$, $[Ir(CO)_3(PPh_3)]^+[PF_6]^-$, $[HPt(PEt_3)_3]^+[PF_6]$, $Rh_2O_3$, $Pd(NO_3)_2$ and $Rh(NO_3)_3$, where R is a hydrocarbyl radical, which may or may not be substituted.

11. A process according to claim 9, in which the ligand contains at least one amine, ammonium, alcohol, carboxylic acid or sulphonate function.

12. A process according to any one of the preceding claims, in which the catalytic composition contains a ligand or a ligand associated with a transition metal compound, selected from the group formed by triphenylphosphine, triphenylphosphine oxide, triphenylphosphite, triethylphosphite, the sodium salt of monosulphonated triphenylphosphine, and the sodium salt of trisulphonated triphenylphosphine.

13. A process according to any one of the preceding claims, in which the concentration of the compound(s) of the transition metal(s) is 1 to 500 mmoles per litre with respect to the ammonium and/or phosphonium salt.

14. A process according to any one of the preceding claims, in which the catalytic composition also contains an organic solvent.

15. A process according to claim 14, in which the solvent is selected from the group formed by aromatic hydrocarbons and aliphatic hydrocarbons.

16. A process according to any one of the preceding claims, in which the olefinically unsaturated compound is selected from the group formed by monoolefins, diolefins, conjugated diolefins, and compound containing one or more unsaturated heteroatoms.

17. A process according to claim 16, in which the monoolefin is selected from the group formed by ethylene, propylene, butene, pentene, hexenes and octenes and the diolefin is selected from the group formed by butadiene and the pentadienes.

18. A process according to any one of claims 8 to 15, in which the olefinically unsaturated compound is a compound carrying a ketone function or a carboxylic acid function.

19. A process according to any one of claims 1 to 16, in which the organic phase containing the reaction products is separated from the polar phase, said polar phase containing at least a portion of the catalyst being at least partially recycled to the hydroformylation reactor.

20. A process according to any one of claims 1 to 18, operating between 30°C and 200°C, at a total pressure in the range 1 Ma to 20 MPa, the ratios of the partial pressure of carbon monoxide to hydrogen being 1:10 to 10:1.

**21.** A process according to any one of claims 1 to 18, in which the aldehydes are selectively produced.

**22.** A process according to any one of claims 1 to 21, in which hydroformylation is carried out at a temperature below to 90°C.